# EUROPÄISCHE PATENTSCHRIFT

(11) **EP 1 022 015 B1**
(45) Veröffentlichungstag und Bekanntmachung des Hinweises auf die Patenterteilung: **04.09.2002**
(21) Anmeldenummer: 99124363.5
(22) Anmeldetag: 07.12.1999
(51) Int. Cl.: A61K 7/13, A61K 7/00

(54) **Verfahren zur Herstellung eines Haarfärbemittels**
PROCESS FOR PREPARING A HAIR DYEING COMPOSITION
PROCEDE DE PREPARATION D'UNE COMPOSITION POUR LA TEINTURE DE CHEVEUX

(30) Priorität: 19.01.1999 DE 19901886
(43) Veröffentlichungstag der Anmeldung: 26.07.2000
(73) Patentinhaber: GOLDWELL GmbH, 64297 Darmstadt (DE)
(72) Erfinder: Fröhling, Beate, Dr., 64625 Bensheim (DE)

(56) Entgegenhaltungen:
- P. HAMEYER: "Herstellungs- und rezepturbedingte Beeinflussung der Konsistenz von O/W-Cremes" SEIFEN, ÖLE, FETTE, WACHSE JOURNAL, Bd. 119, Nr. 13, 1993, Seiten 759-774, XP000398731 Augsburg, DE
- K. F. DE POLO: "A Short Textbook of Cosmetology" 1998 , H. ZIOLKOWSKY GMBH , AUGSBURG, DEUTSCHLAND XP002164678 Seiten 269-289 * Seite 269 - Seite 271 * * Abbildungen 8-18 * * Seite 277 *

## Beschreibung

Die vorliegende Erfindung betrifft ein Verfahren zur Herstellung einer Haarfärbemittels auf Basis einer feinen wäßrigen Emulsion, enthaltend mindestens ein Oxidationsfarbstoff-Vorprodukt.

Permanente Haarfärbemittel auf Basis von Oxidationsfarbstoffen erfreuen sich einer weiten Verbreitung. Ihre Anwendung erfolgt in der Regel dergestalt, daß eine flüssige, zumeist als wäßrige Emulsion vorliegende, mindestens ein Oxidationsfarbstoff-Vorprodukt, im allgemeinen mindestens eine Entwickler- und mindestens eine Kupplersubstanz, enthaltende Zusammensetzung unmittelbar vor der Anwendung mit einer Peroxid enthaltenden Zusammensetzung vermischt, und die Mischung auf das Haar aufgebracht wird.

Die Herstellung dieser Emulsionen geschieht durch Heißemulgieren der Bestandteile und darauffolgendes Abkühlen, was naturgemäß energie- und zeitintensiv ist und auch nicht immer zu stabilen Emulsionen führt.

Die Erfindung geht daher von der Aufgabenstellung aus, diese Probleme zu vermeiden und ein Verfahren zur Herstellung von Haarfärbeemulsionen zur Verfügung zu stellen, das zeitund energiesparend arbeitet, wobei die erhaltenen Emulsionen auch eine gute Vermischbarkeit mit der Oxidationsmittel-Zusammensetzung, d.h. im Regelfall wäßrigem Wasserstoffperoxid, gewährleisten, was wiederum zu einer gleichmäßigen Verteilung des fertigen Färbemittels auf dem Haar und damit zu einer guten Färbeleistung führt.

Die erfindungsgemäße Lösung dieser Aufgabe besteht in einem Verfahren zur Herstellung eines Haarfärbemittels in Form einer stabilen wäßrigen Emulsion, das dadurch gekennzeichnet ist, daß
a) zunächst die wasserlösliche Tenside, Salze und gegebenenfalls sonstige wasserlösliche Zusätze, insbesondere Oxidationsfarbstoff-Vorprodukte, enthaltende Wasserphase auf 50 bis 90°C; insbesondere etwa 60 bis 80 °C; erhitzt wird, anschließend
b) unter Rühren bei Aufrechterhaltung dieser Temperatur die Fettphasenkomponenten, deren Schmelzpunkt zwischen 20°C und der jeweils angewandten Temperatur zwischen 50 und 90°C liegt, zugesetzt und solange bei dieser Temperatur gerührt wird, bis sie vollständig geschmolzen sind; und
c) der so erhaltenen Zusammensetzung die restlichen flüssigen Fettphasen-Bestandteile ohne Aufrechterhaltung der erhöhten Temperatur zugesetzt werden.

Dieses Verfahren stellt gegenüber dem bislang benutzten Heißemulgier-Verfahren eine wesentlich zeit- und energiesparende und damit auch kostengünstige Alternative dar, da durchschnittlich nur noch etwa 30 % der Fettphase und ca. 80 % der gesamten Emulsion zunächst erhitzt und später wieder abgekühlt werden müssen, und zusätzlich auch noch eine schnellere Abkühlung durch die separate Zugabe der flüssigen Fettphasenstoffe zu der Vermischung erfolgt.

Das bevorzugte Gewichtsverhältnis Fettphase zu Wasserphase der gebrauchsfertigen Färbeemulsion liegt bei etwa 20 zu 80 bis 50 zu 50, insbesondere bei etwa 25 zu 75 bis 40 zu 60; das bevorzugte Gewichtsverhältnis von festen zu flüssigen Fettphasenbestandteilen bei etwa 3 zu 1 bis 1 zu 3, vorzugsweise etwa 2 zu 1 bis 1 zu 2.

Bevorzugte flüssige Stoffe der Fettphase sind beispielsweise bei Raumtemperatur, d.h. etwa 20 bis 25 °C, flüssige Fettsäuren, insbesondere Ölsäure.
Die bevorzugte Menge dieser Fettsäuren liegt bei etwa 5 bis 15, insbesondere bei etwa 6 bis 12 Gew.-%, berechnet auf die Gesamtzusammensetzung.

Weitere bevorzugte flüssige Bestandteile der Fettphase sind C₁₀-C₂₂-Fettalkoholethoxylate, vorzugsweise Lauryl-, Kokos- und Oleylalkohol- Ethylenoxid-Kondensate in einer Menge von etwa 5 bis 25, insbesondere etwa 10 bis 20, beispielsweise etwa 15 Gew.-%, berechnet auf die Gesamtzusammensetzung.

Auch flüssige Zuckerfettsäureester wie Saccharose- und Glucosediester, insbesondere Dioleate, können mitverwendet werden, beispielsweise Glucose- und Methylglucosedioleat in einer Menge von etwa 5 bis 25, z.B. etwa 10 bis 15 Gew.-% der Gesamtzusammensetzung.

Weitere mitverwendbare Bestandteile sind flüssige Fettalkohole wie Oleylalkohol und Polyole, z.B. Glycerin und Propylenglycol.

Geeignete feste Fettphasenkomponenten, deren Schmelzpunkt zwischen 20 °C, vorzugsweise 25 bis 30 °C, und dem bei ihrem Einrühren in feinverteilter Form in die Wasserphase bei 50 bis 90 °C eingestelltem Temperaturbereich liegt, sind insbesondere Emulgatoren und Fettkörper, z.B. entsprechende höhere Fettalkohole wie Myristylalkohol, Cetylalkohol, Stearylalkohol und Fettalkoholgemische, höhere Fettsäureester wie Glycerin-, Ethandiol- und Propandiolfettsäureester, beispielsweise Ethandiolmono- und -distearat, 1,2-Popylenglykolmono- und -distearat sowie Glycerylstearat.

Weitere geeignete feste Fettphasenbestandteile sind höhere Fettsäuremono- und - dialkanolamide wie Cocosfettsäure- und Stearinsäuremonoethanolamid. Vorzugsweise werden Gemische dieser Substanzen mit oberflächenaktiven Eigenschaften eingesetzt.

Die Viskosität der erfindungsgemäß hergestellten Haarfarbeemelsionen liegt vorzugsweise bei etwa 5.000 und 30.000, insbesondere bei etwa 10.000 bis 25.000, beispielsweise bei etwa 15.000 bis 20.000 mPa•s, gemessen bei 20 °C im Brookfield-Viskosimeter RVT.

Die Wasserphase kann wasserlösliche Emulgatoren enthalten. Als solche können insbesondere anionische Tenside verwendet werden.

Geeignete anionaktive Tenside im Rahmen der Erfindung sind insbesondere in einer Menge von etwa 0,25 bis etwa 5 Gew.-%, vorzugsweise etwa 0,4 bis 2,5 Gew.-% der Gesamtzusammensetzung (einsatzfertige Emulsion) enthalten.

Dabei handelt es sich um solche vom Sulfat-, Sulfonat-, Carboxylat- und Alkylphosphat-Typ, vor allem natürlich diejenigen, die in Haarbehandlungsmitteln üblicherweise zum Einsatz gelangen, insbesondere die bekannten C₁₀-C₁₈-Alkylsulfate und die entsprechenden Ethersulfate, beispielsweise C₁₂-C₁₄-Alkylethersulfate, Laurylethersulfat, insbesondere mit 1 bis 4 Ethylenoxidgruppen im Molekül, Acylaminocarbonsäuren wie Lauroylsarkosinat und -glutamat, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und Alkalisalze von Sulfobernsteinsäurehalbestern, beispielsweise das Dinatriumsalz des Monooctylsulfosuccinats, und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 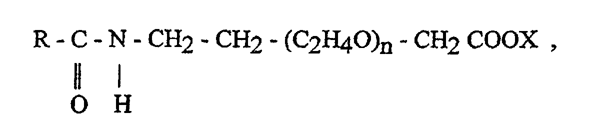 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO®" und "AKYPO-SOFT®".
Auch C₈-C₂₀-Acylisethionate können, allein oder im Gemisch mit anderen Tensiden, eingesetzt werden, ebenso Sulfofettsäuren und deren Ester.

Gegebenenfalls können auch amphotere bzw. zwitterionische Tenside als wasserlösliche Emulgatoren eingesetzt werden, insbesondere im Gemisch mit anionaktiven Tensiden, wobei die Gesamtmenge vorzugsweise bei etwa 0,25 bis 5, insbesondere etwa 0,5 bis 2,5 Gew.-% der Färbeemulsion liegen soll.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

Im einzelnen können Betaine der Struktur 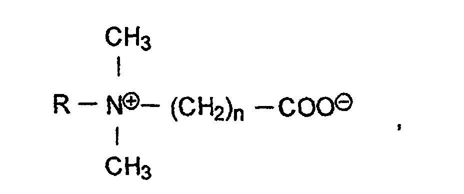 und 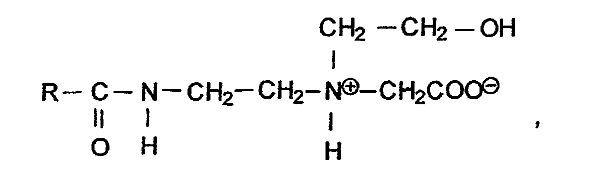 wobei R eine C₈-C₁₈-Alkylgruppe und n 1 bis 3 bedeuten,
Sulfobetaine der Struktur 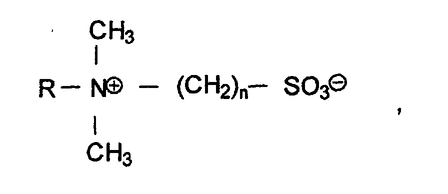 wobei R eine C₈-C₁₈-Alkylgruppe und n 1 bis 3 bedeuten,
und Amidoalkylbetaine der Struktur 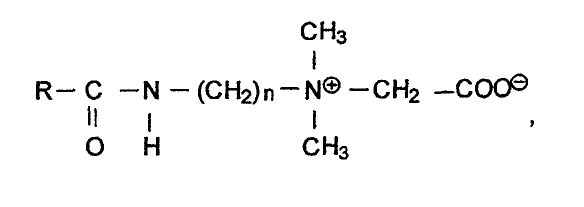 wobei R eine C₈-C₁₈-Alkylgruppe und n 1 bis 3 bedeuten, eingesetzt werden.

Auch die Verwendung nichtionischer wasserlöslicher Tenside, z.B. von C₈-C₁₈-Alkylpolyglucosiden mit einem Polymerisationsgrad von 1 bis 5, ist in den genannten Mengen alleine oder im Gemisch mit anionischen und/oder amphoteren bzw. zwitterionischen oberflächenaktiven Substanzen möglich.

### Auch Aminoxide sind einsetzbar.

Weitere geeignete Tenside sind auch kationische Tenside wie die bekannten quaternären Ammoniumverbindungen mit einer oder zwei Alkyl- bzw. Alkenylgruppen mit 10 bis 22 Kohlenstoffatomen im Molekül, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumbromid, Behenyltrimoniumchlorid, Stearyltrimethylammoniumchlorid, Dimethylstearylbenzylammoniumchlorid, Benzyltetradecyldimethylammoniumchlorid, Dimethyl- dihydriertes-Talgammoniumchlorid, Laurylpyridiniumchlorid, Lauryldimethylbenzylammoniumchlorid, Lauryltrimethylammonuiumchlorid, Tris-(oligooxyethyl)alkylammoniumphosphat, Cetylpyridiniumchlorid, etc.

Grundsätzlich sind alle quaternären Ammoniumverbindungen geeignet, die im CTFA International Cosmetic Ingredient Dictionary unter der Bezeichnung " Quaternium" aufgeführt sind.

Die erfindungsgemäße Haarfärbeemulsion enthält mindestens ein Oxidationsfarbstoffvorprodukt, zweckmäßigerweise ein Gemisch aus mindestens einer Entwickler- und mindestens einer Kupplersubstanz.

Diese sind an sich bekannt und beispielsweise in der Monographie von K. Schrader, Grundlagen und Rezepturen der Kosmetika, 2. Aufl. (1989), S. 784-799, beschrieben.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2'-Hydroxyethylamino)-5-aminotoluol und 1-Amino-4-bis-(2'-hydroxyethyl)-aminobenzol bzw. deren wasserlösliche Salze; beispielhafte Kupplersubstanzen sind Resorcin, 2-Methylresorcin, 4-Chlorresorcin, 2-Amino-4-chlorphenol,4-(N-Methyl)-aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodiphenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'hydroxyethylamino)-benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in der erfindungsgemäßen Farbemulsion kann jeweils etwa 0,1 bis etwa 5 Gew.-%, je nach gewünschter Färbung, betragen.

Diese Oxidationsfarbstoffvorprodukte sind zweckmäßigerweise bereits in der wäßrigen Phase enthalten; sie können jedoch auch, falls erwünscht, dem Fertigprodukt zugesetzt werden.

Die erfindungsgemäß hergestellten Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäß hergestellten Haarfärbemittel können die in solchen Mitteln üblichen Grund- und Zusatzstoffe, Konditioniermittel, Stabilisatoren, Fette und Öle, Verdickungsmittel, Komplexbildner, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind.

Die erfindungsgemäß hergestellten Färbeemulsionen weisen vorzugsweise einen im alkalischen Bereich liegenden pH-Wert, insbesondere zwischen etwa 8 und etwa 12,5, vorzugsweise zwischen 8,5 und 11, auf.

Zur Applikation wird die erfindungsgemäß hergestellte Oxidationsfarbstoff-Vorprodukt-Emulsion mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d.h. nach Vermischung mit Peroxid, kann sowohl im schwach sauren, d.h. einem Bereich von 5,5 bis 6,9, im neutralen als auch im alkalischen Bereich, d.h. zwischen pH 7,1 und 10 liegen.

Im folgenden wird die Erfindung anhand von Ausführungsbeispielen erläutert.

### Beispiel 1

Eine wäßrige Phase, bestehend aus:

| | |
|---|---|
| Ammoniak, 25 %-ig | 92,00 (Gew.-Teilen) |
| Pyrogenes Siliciumdioxid | 1,00 |
| Natriumlaurylsulfat | 5,00 |
| EDTA (Trinatriumsalz) | 2,00 |
| Ammoniumchlorid | 5,00 |
| Natriumsulfit | 10,00 |
| Ascorbinsäure | 5,00 |
| Kationisches Pflanzeneiweißhydrolysat | 4,50 |
| Panthenol | 6,00 |
| Hopfenextrakt | 5,00 |
| Parfum | 3,60 |
| und den Oxidationsfarbstoff-Vorprodukten | |
| p-Toluylendiaminsulfat | 5,40 |
| Resorcin | 0,59 |
| 4-Chlorresorcin | 1,65 |
| 3-Aminophenol | 0,25 |
| 3-Hydroxy-4-methylanilin | 0,05 |
| und 6-Hydroxy-2,4,5-triaminopyrimidinsulfat | 0,10 |
| sowie | |
| Wasser | 595,95 |

wurde auf etwa 65 °C erhitzt und in diese heiße Lösung unter Rühren das folgende Gemisch aus festen Fettbestandteilen zugesetzt, die sich durch Schmelzen in der heißen Wasserphase verteilen:

| | |
|---|---|
| Cetylstearylalkohol/Natriumcetylstearylsulfat (9:1) | 120,00 (Gew.-Teile) |
| Stearinsäuremonoethanolamid | 23,00 |
| Cocosfettsäuremonoethanolamid | 23,00 |
| Propylenglycolstearat (Tegin® P) | 6,00 |

Anschließend wurden noch, unter Rühren und bei abgestellter Heizung, die folgenden flüssigen Fettphasenbestandteile zugesetzt:

| | |
|---|---|
| Oleth-5 | 50,00 (Gew.-Teile) |
| Ölsäure | 25,00 |
| 1,2-Propandiol | 10,00 |

Es wurde eine feinteilige, stabile Emulsion erhalten, die bei der Applikation mit einer verdünnten Wasserstoffperoxid-Lösung oder -Emulsion eine dauerhafte mittelblonde Haarfärbung ergab.

### Beispiel 2

Zu 680 Gewichtsteilen der in Beispiel 1 beschriebenen Wasserphase wurden bei 60 °C unter Rühren 100 Gewichtsteile eines pulverförmigen Gemischs aus 70 Gewichtsteilen Glycoldistearat und 30 Gewichtsteilen Glycerylstearat eingebracht und anschließend, unter weiterem Rühren und bei abgestellter Heizung noch 220 Gewichtsteile eines flüssigen Gemisches aus 120 Gewichts-teilen Laureth-2 und 100 Gewichtsteilen Ölsäure zugesetzt.

Es wurde wiederum eine stabile, feinteilige Farbemulsion erhalten, die sich mit flüssigen Wasserstoffperoxid-Zubereitungen gut zu einer Färbezubereitung verarbeiten ließ, die auf dem Haar eine langanhaltende Mittelblondfärbung ergab.

## Patentansprüche

1. Verfahren zur Herstellung eines Haarfärbemittels in Form einer stabilen wäßrigen Emulsion, **dadurch gekennzeichnet, daß**
a) zunächst die wasserlösliche Tenside, Salze und gegebenenfalls sonstige wasserlösliche Zusätze enthaltende Wasserphase auf 50 bis 90°C erhitzt, und anschließend
b) unter Rühren bei Aufrechterhaltung dieser Temperatur die festen Fettphasenkomponenten, deren Schmelzpunkt zwischen 20°C und der in Verfahrensstufe a) jeweils angewandten Temperatur zwischen 50 und 90°C liegt, zugesetzt und solange bei dieser Temperatur gerührt werden, bis diese vollständig geschmolzen sind; und
c) der so erhaltenen Zusammensetzung die restlichen flüssigen Fettphasen-Bestandteile zugesetzt werden.

2. Verfahren nach Anspruch 1, **dadurch gekennzeichnet, daß** die Temperatur nach a) bei etwa 60 bis 80 °C liegt.

3. Verfahren nach Anspruch 1 oder 2, **dadurch gekennzeichnet, daß** die Wasserphase die Oxidationsfarbstoff-Vorprodukte enthält.

## Claims

1. Process for the preparation of a stable, aqueous emulsion for the dyeing of human hair, wherein
a) first the water phase containing water-soluble surfactants, salts and optionally other water-soluble additives is heated to 50° and 90°C and, subsequently,
b) while maintaining this temperature, those solid fatty components having a melting point between 20°C and the temperature applied in step a) between 50° and 90°C are added, under stirring at this temperature until they are completely melted in the mixture, and,
c) the remaining liquid fatty-phase components are added to the composition thus obtained without any further heating.

2. Process according to claim 1, wherein the temperature according to a) ranges from about 60 ° to 80°C.

3. Process according to claims 1 or 2, wherein the water phase comprises at least one oxidation dyestuff precursor.

## Revendications

1. Procédé de préparation d'un agent de coloration des cheveux présentant la forme d'une émulsion aqueuse stable, **caractérisé en ce que** :
a) la phase aqueuse contenant les agents tensioactifs solubles dans l'eau, les sels et éventuellement d'autres additifs solubles dans l'eau est d'abord chauffée entre 50 et 90° C, et **en ce qu'**ensuite
b) sous agitation et en maintenant cette température, les composants solides de la phase grasse dont le point de fusion est situé entre 20° C et la température particulière utilisée pendant l'étape a) du procédé comprise entre 50 et 90° C sont ajoutés et sont brassés à cette température jusqu'à être complètement fondus, et **en ce que**
c) les autres composants liquides de la phase grasse sont ajoutés à la composition ainsi obtenue.

2. Procédé selon la revendication 1, **caractérisé en ce que** la température de l'étape a) est comprise entre 60 et 80° c.

3. Procédé selon la revendication 1 ou 2, **caractérisé en ce que** la phase aqueuse contient les précurseurs de colorants d'oxydation.
